Europäisches Patentamt

(19) European Patent Office

Office européen des brevets

(11) EP 1 257 301 B1

(12) EUROPEAN PATENT SPECIFICATION

(45) Date of publication and mention
of the grant of the patent:
06.05.2004 Bulletin 2004/19

(51) Int Cl.⁷: A61L 2/20, A61L 2/18

(21) Application number: 01904161.5

(86) International application number:
PCT/GB2001/000568

(22) Date of filing: 12.02.2001

(87) International publication number:
WO 2001/058500 (16.08.2001 Gazette 2001/33)

(54) **MEDICAL DEVICE DISINFECTION**

DESINFEKTION VON MEDIZINISCHEN GERÄTEN

DESINFECTION DE DISPOSITIF MEDICAL

(84) Designated Contracting States:
AT BE CH CY DE DK ES FI FR GB GR IE IT LI LU
MC NL PT SE TR

(30) Priority: 10.02.2000 GB 0003110

(43) Date of publication of application:
20.11.2002 Bulletin 2002/47

(73) Proprietor: Bioquell Medical Limited
Weston-Super-Mare, Somerset BS24 9BP (GB)

(72) Inventors:
• DAINTREE, Andrew
Lymphsam BS24 0EN (GB)

• CURTIS, Beverley
Taunton TA3 6HA (GB)

(74) Representative: Bayliss, Geoffrey Cyril et al
BOULT WADE TENNANT,
Verulam Gardens
70 Gray's Inn Road
London WC1X 8BT (GB)

(56) References cited:
EP-A- 0 773 031          WO-A-97/40860
US-A- 5 443 801          US-A- 5 520 893
US-A- 5 853 014

## Description

This invention relates to the disinfection of medical equipment such as endoscopes, bed pans and other health care equipment.

Medical devices and in particular endoscopes have historically been disinfected by either heat or chemicals. Current disinfection of endoscopes is carried out by two methods; one a cold process, and the other a heated process.

### i) Cold Process

This is normally used when the endoscopes cannot be disinfected by using heat, i.e. most flexible endoscopes. The endoscopes are manually cleaned and then put into a washer disinfector for an automatic process. This process gives the scopes a pre-wash, a wash with a disinfectant, and a final rinse with water. The disinfectant wash allows a contact time dependent on the manufacturer of the disinfectant, e.g. Cidex (Johnson and Johnson), Nu-Cidex (Johnson and Johnson), Gigasept (Schule and Mayer).

Cold processing allows a batch of disinfectant to be re-used, the number of cycles dependent on the washer disinfector and the level of dilution taking place.

Once this number of cycles is completed, the batch of disinfectant is dumped to waste and the machine re-charged with a fresh bash.

### ii) **Hot Process**

Some endoscopes (mainly rigid ones) can be processed in a normal sterilising autoclave at 120-130°C. For endoscopes such as flexible ones that cannot withstand this temperature, there are a range of washer disinfectors that disinfect by heating to a lower temperature of 50-55°C.

This process gives the scopes a pre-wash, a heated wash with a small amount of disinfectant, and then a final rinse with water. The heated wash takes a small amount of concentrated disinfectant, and by heating to 50-55°C causes the chemical to vaporise and thus provide the efficiency required.

This process normally uses gluteraldehyde as the disinfectant, and the small amount used each time is a single use. This process tends to have longer cycle times than cold processing.

The heated process is more prevalent in Europe, while cold processing is utilised in the UK and US.

The method of the invention also provides an alternative to the use of steam.

Ozonated water is widely used to kill microbiological organisms. However, when generating and dissolving ozone in water it is usual to expect levels of under 1 ppm. We have found that we are not able to disinfect medical devices to the required standard or within an acceptable time period using such levels of ozone concentration. Effective disinfection can only be achieved with a precise combination of flow over and through the device, ozone levels, and time.

The criteria for disinfection of the endoscopes have been developed by Dr. J Babb of the Hospital Infection Research Laboratory (HIRL) at City Hospital NHS Trust, Birmingham, as described later and is key to the validation of the process. The process fulfils the HIRL test criteria for endoscope washer disinfectors, i.e. mean $\log_{10}$ reduction >6 (99.9999%) with no individual reduction <5 (see Appendix 3). Although external validation of the process can be undertaken, it is impractical to undertake on a daily basis. Within the process we have been able to measure the ozone levels at the inlet and outlet of the process. This has allowed us to calculate how long the process needs to run to give the required disinfection. As ozone concentration is depleted on contact with microbiological organisms, if the inlet and outlet levels are identical there is minimal microbiological organisms remaining. As microbiological organisms levels have to be very low to validate the unit for a predetermined time after equilibrium is reached.

Thus this invention relates to ozonated water as a substitute for the traditional chemical method of disinfection. Although the development and validation has been undertaken on endoscopes, the process and technology is relevant to many medical devices.

US-A 5520893 discloses an apparatus for sterilising articles with ozone. Medical instruments, including stainless steel, plastic tubing and the like are sterilised in a portable apparatus that provides a low volume, high pressure fluid of continuously circulating water containing about 2 to 6 ppm of ozone.

US-A-5443801 discloses an endoscope cleaner-steriliser apparatus comprising a capsule for holding the endoscope, the capsule having a re-sealable entry port for depositing the endoscope into the capsule and a flexible peristaltic zone which is deformable under pressure. The diaphragm encircles the endoscope within the capsule and provides a partial seal within the capsule. A liquid is contained within the capsule and an agitator-has a tray for accepting the capsule. Pressure means are provided for applying pressure to the flexible peristaltic zone of the capsule and a motor is provided for oscillating the pressure means over the section of the flexible peristaltic zone. The agitator includes a means for generating a ozone gas and means for selectively communicating the ozone gas into the capsule via the inlet port.

EP-A-773031 discloses a rapid purifying method using ozonised water of high concentration. Verifying organisms such as protozoa and plankton, or bacteria or virus are subjected to the action of ozonised water for killing, or oils, fat, resins or other contaminants adhered to objects which are subjected to the action of ozonised water for exfoliation from the objects.

The invention provides a method of disinfecting medical equipment comprising the steps of causing ozonated water to flow over the surfaces of the equipment at a predetermined concentration and flow rate for a certain time and monitoring the concentration of ozone in the water leaving the equipment and terminating the flow when the concentration of ozone leaving the equipment is substantially the same as that being delivered to the equipment. Thus the rinse water produced does not contain active sanitants.

The following is a description of some specific embodiments of the invention, reference being made to the accompanying drawings, in which:

Figure 1 is a schematic diagram showing the apparatus used for carrying the disinfection of medical equipment such as endoscopes; and

Figure 2 shows a number of different scopes to which the cleaning process is applicable.

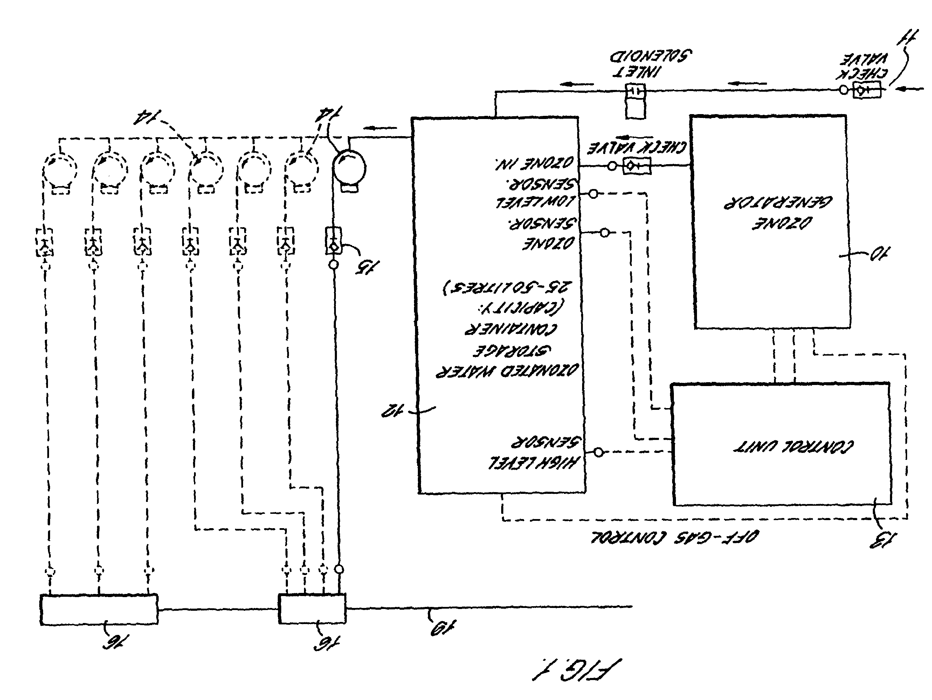

Figure 1 shows a unit based around an electrochemical generator stack 10, where Hydrogen (H) and Ozone ($O^3$) are generated. The stack is fed by a dedicated de-ionised water supply 11 at a pressure of one bar, to maintain the integrity and efficiency of the cells and the long-term quality of the feed water. Power for the stack is supplied from a variable DC supply (not shown). There is also a battery back-up system (not shown) to support the cell in the event of a power failure.

Hydrogen gas is re-absorbed and/or catalytically converted. Ozone is supplied under pressure to a contactor 12 containing 25-50 litres of filtered water via a diffuser block. This allows the ozone gas to bubble into the water to produce a high concentration solution (typically at least 4 ppm, for example 6+ppm). The level of water in the contactor is controlled and filled through solenoid valves, the operations being initiated by a micro-processor operated control unit 13 through software instructions. Ozone concentration levels are constantly monitored to ensure correct values.

Any excess ozone off-gas is collected at the top of the contactor and passed to a destruct column, where it is processed through an absorber. When operating at full capacity the cell produces perceptible heat, and so water used in the cell for electrolysing is cooled with a heat exchanger and refrigeration plant.

To disinfect effectively an endoscope, ozonated water needs to be pumped through all the internal channels of the scope at a flow rate and concentration level sufficient to kill organisms that may remain after a manual clean has taken place. The water is supplied at ambient temperature.

In our testing we have found these to be concentration level of at least 4ppm, preferably about 6ppm and not more than 15ppm, and a flow rate that equates to 2.2 L/min. These parameters need to be applied for a minimum period of 10 minutes and a maximum of 15 minutes to ensure all internal channels of a normal endoscope have been disinfected.

Ozonated water is supplied from the contactor to a supply pump 14 having connectors 15 for coupling to the individual endoscope channels 16. Spent ozonated water is directed to waste via the distal end of the endoscope 19.

During testing and development a method was devised that determined whether ozone has achieved the intended uses. With a known concentration of ozonated water entering a test sample contaminated with "Pseudomonas aeruginosa NCTC 6749" of a known value, a second sensor was used to monitor the water output from the test equipment. When the exit concentration level rose to match the known input, it was assumed that by then ozone had killed any remaining organisms. Unlike called for in present claim 1, the flow of ozonated water was continued for a further 5 minutes after that equilibrium was reached. Sterile water samples were taken and cultured to established protocols and showed that the method had achieved the necessary kill rates, to be declared a process disinfectant.

Previous tests were conducted that used ozone concentrations that varied from 0.1-18ppm, and flow rates as low as 400ml/min. Contact times also varied from 5 minutes to as much as 25 minutes, but in all cases the kill rates achieved were inferior to those reached when the optimised settings previously stated were used.

**KEY POINTS**

*    Safe operating media - chemical disinfectants are sensitising agents and are possibly tetragenic.
*    Cold Process.
*    One Shot Process.
*    Process validated.
*    Closed loop system - ozone levels monitored at discharge.

* Critical parameters of ozone concentration, flow discharge rates, and time established.
* Residue free disinfectant.

**TEST METHOD**

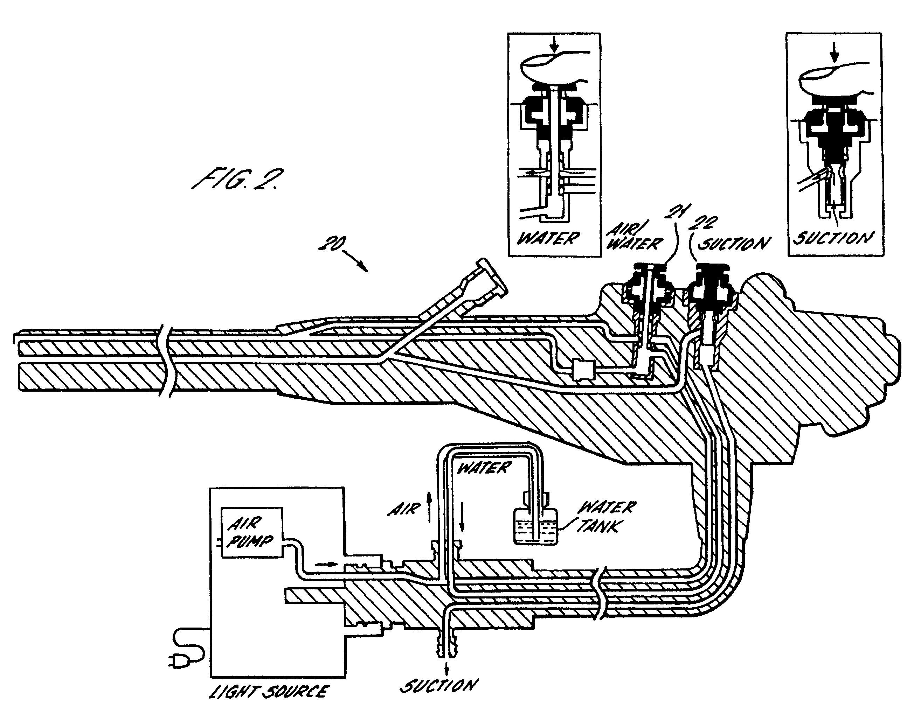

The biopsy and suction channels of an Olympus gastroscope 20 (Type GIF Q10) shown in Figure 2 were contaminated having removed the air/water and suction valves 21,22 with an overnight broth culture of Ps. *aeruginosa* NCTC 6749 enriched with 10% horse serum. The-instrument was left to drain / dry for 10 minutes at room temperature before sampling, i.e. pre-disinfection count or processing and sampling, ie. post-disinfection count. The endoscope was recontaminated prior to each test cycle. After processing in an endoscope washer disinfector by coupling supply and return conduits to the air/water and section valve parts, the endoscope channels were sampled to detect surviving test bacteria. This was done by flushing 10 ml of sterile water through the channel lumens and collecting the washings in a sterile container at the distal tip. These were diluted and plated onto typtone soya agar plates, which were incubated at 37°C for 18 hours. The number of colony forming units of the test organism were enumerated and counts transposed to the $log_{10}$ system. The log reduction (RF) were calculated for each cycle, i.e.:

$$Log_{10} \text{ pre-disinfection count - } log_{10} \text{ post-disinfection count}$$

$$= log_{10} \text{ reduction (RF)}$$

It is normal to use a pre-disinfection count of 8 $log_{10}$ contamination and aim for a post-disinfection count of less than 2 $log_{10}$, giving a $log_{10}$ reduction of 6.

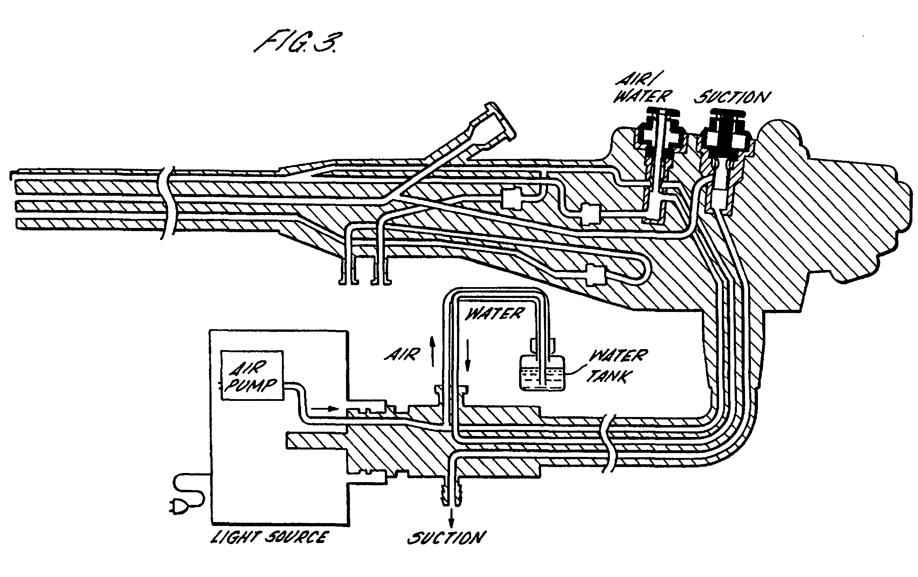

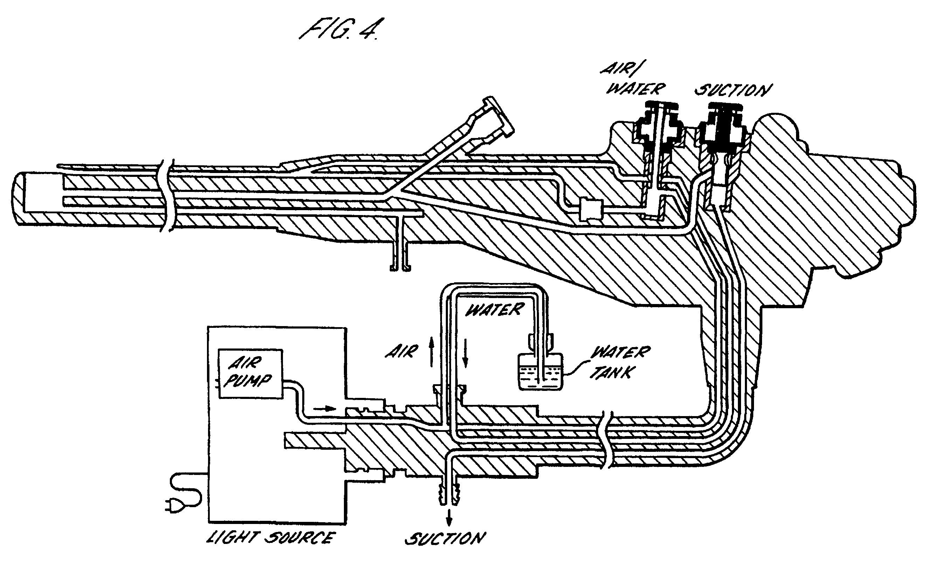

Similar methods are used for colonoscopes (see Figure 3) and duadenoscopes (see Figure 4).

**Definition of Disinfection**

PHLS - Chemical Disinfection in Hospitals

**Definitions**

**Disinfection:** A process used to reduce the number of micro-organisms but not usually of bacterial spores; the process does not necessarily kill or remove all micro-organisms, but reduces their number of a level which is not harmful to health.' The term is applicable to the treatment of inanimate objects and materials and may also be applied to the treatment of the skin, mucous membranes and other body tissues and cavities.

**ENDOSCOPE TESTS**

| $Log_{10}$ reductions in test bacteria from the biopsy and suction channels of two endoscopes using ozonated water for 10 minutes | | | | |
|---|---|---|---|---|
| **Contact time** | **Water (control)** | | **Ozonated water** | |
| | **Biopsy channel** | **Suction channel** | **Biopsy channel** | **Suction channel** |
| **Olympus GIF Q10** Pre-treatment | 8.58 | 8.41 | 8.43 | 8.54 |
| Post-treatment | | | | |
| Cycle 1 | 4.74 | 3.63 | 5.95 | 6.33 |
| Cycle 2 | 3.70 | 4.46 | 6.04 | 6.39 |
| Cycle 3 | 4.17 | 4.45 | 6.17 | 6.39 |
| Mean $log_{10}$ RF | 4.20 | 4.09 | 6.05 | 6.33 |
| Fujinon Colonoscope Pre-treatment | 8.59 | 8.69 | 8.59 | 8.69 |

(continued)

| Log$_{10}$ reductions in test bacteria from the biopsy and suction channels of two endoscopes using ozonated water for 10 minutes | | | | |
|---|---|---|---|---|
| Contact time | Water (control) | | Ozonated water | |
| | Biopsy channel | Suction channel | Biopsy channel | Suction channel |
| Post-treatment | | | | |
| Cycle 1 | 5.48 | 5.28 | 5.75 | 5.99 |
| Cycle 2 | 5.55 | 5.46 | 6.44 | 6.61 |
| Mean log$_{10}$ RF | 5.52 | 5.37 | 6.10 | 6.30 |

## Claims

1. A method of disinfecting medical equipment comprising the steps of causing ozonated water to flow over the surfaces of the equipment at a predetermined concentration and flow rate for a certain time, **characterised in that** the concentration of ozone in the water leaving the equipment is monitored and the flow of ozonated water is terminated when the concentration of ozone leaving the equipment is substantially the same as that being delivered to the equipment.

2. A method as claimed in claim 1, **characterised in that** the equipment is subjected to a manual washing process prior to disinfection by ozonated water.

3. A method as claimed in claim 1 or claim 2, **characterised in that** the ozonated water has a concentration of at least 5 ppm and not more than 15 ppm.

4. A method as claimed in claim 3, **characterised in that** the ozonated water has a concentration of 15 ppm.

5. A method as claimed in claim 3 or claim 4, **characterised in that** the flow rate of ozonated water over the surfaces of the equipment is approximately 2.2 litres per minute.

6. A method as claimed in any of the preceding claims, **characterised in that** the equipment has an internal channel or channels, wherein ozonated water is'delivered over the outer surfaces of the equipment and through the internal channels of the equipment.

7. A method as claimed in any of the preceding claims, **characterised in that** the equipment is an endoscope having one or more internal channels through which said ozonated water is caused to flow.

8. A method as claimed in any of the preceding claims, **characterised in that** the equipment is subjected to a final rinse in water following said disinfecting process.

## Patentansprüche

1. Verfahren zum Desinfizieren medizinischer Geräte, umfassend die Schritte, dass man für eine bestimmte Zeit ozonisiertes Wasser bei einer vorbestimmten Konzentration und Fließgeschwindigkeit über die Oberflächen der Geräte fließen lässt, **dadurch gekennzeichnet, dass** man die Ozonkonzentration im die Geräte verlassenden Wasser überwacht und den Fluss des ozonisierten Wassers beendet, wenn die die Geräte verlassende Ozonkonzentration im Wesentlichen dieselbe ist wie die den Geräten zugeführte.

2. Verfahren nach Anspruch 1, **dadurch gekennzeichnet, dass** man die Geräte vor der Desinfektion mit ozonisiertem Wasser einem manuellen Waschverfahren unterzieht.

3. Verfahren nach Anspruch 1 oder Anspruch 2, **dadurch gekennzeichnet, dass** das ozonisierte Wasser eine Konzentration von mindestens 5 ppm und nicht mehr als 15 ppm aufweist.

**4.** Verfahren nach Anspruch 3, **dadurch gekennzeichnet, dass** das ozonisierte Wasser eine Konzentration von 15 ppm aufweist.

**5.** Verfahren nach Anspruch 3 oder Anspruch 4, **dadurch gekennzeichnet, dass** die Fließgeschwindigkeit des ozonisierten Wassers über die Oberflächen der Geräte ungefähr 2,2 Liter pro Minute beträgt.

**6.** Verfahren nach einem der vorhergehenden Ansprüche, **dadurch gekennzeichnet, dass** die Geräte einen internen Kanal oder Kanäle aufweisen, wobei das ozonisierte Wasser über die äußeren Oberflächen der Geräte und durch die internen Kanäle der Geräte zugeführt wird.

**7.** Verfahren nach einem der vorhergehenden Ansprüche, **dadurch gekennzeichnet, dass** es sich bei den Geräten um ein Endoskop mit einem oder mehreren internen Kanälen handelt, durch welche man das ozonisierte Wasser fließen lässt.

**8.** Verfahren nach einem der vorhergehenden Ansprüche, **dadurch gekennzeichnet, dass** man nach dem Desinfektionsverfahren die Geräte einer letzten Spülung in Wasser unterzieht.


**Revendications**

**1.** Procédé de désinfection de matériel médical consistant à faire circuler de l'eau ozonée sur les surfaces du matériel à une concentration et à un débit d'écoulement prédéterminés pendant un certain temps, **caractérisé en ce que** la concentration d'ozone dans l'eau quittant le matériel est contrôlée et l'écoulement d'eau ozonée est arrêtée lorsque la concentration d'ozone quittant le matériel est sensiblement égale à celle délivrée au matériel.

**2.** Procédé selon la revendication 1, **caractérisé en ce que** le matériel est soumis à un processus de lavage manuel avant la désinfection par l'eau ozonée.

**3.** Procédé selon la revendication 1 ou la revendication 2, **caractérisé en ce que** l'eau ozonée a une concentration d'au moins 5 ppm et ne dépassant pas 15 ppm.

**4.** Procédé selon la revendication 3, **caractérisé en ce que** l'eau ozonée a une concentration de 15 ppm.

**5.** Procédé selon la revendication 3 ou la revendication 4, **caractérisé en ce que** le débit d'écoulement d'eau ozonée sur les surfaces du matériel est d'environ 2,2 litres par minute.

**6.** Procédé selon l'une quelconque des revendications précédentes, **caractérisé en ce que** le matériel comporte un canal ou des canaux internes, dans lequel l'eau ozonée est délivrée sur les surfaces extérieures du matériel et dans les canaux interne du matériel.

**7.** Procédé selon l'une quelconque des revendications précédentes, **caractérisé en ce que** le matériel est un endoscope ayant un ou plusieurs canaux internes dans lesquels ladite eau ozonée est amenée à s'écouler.

**8.** Procédé selon l'une quelconque des revendications précédentes, **caractérisé en ce que** le matériel est soumis à un rinçage final dans de l'eau après ledit processus de désinfection.

FIG. 1.

OFF-GAS CONTROL

13
CONTROL UNIT

10
OZONE GENERATOR

12
OZONATED WATER STORAGE CONTAINER (CAPICITY: 25-50 LITRES)

HIGH LEVEL SENSOR

OZONE SENSOR.

LOW LEVEL SENSOR.

OZONE IN.

CHECK VALVE

INLET SOLENOID

11
CHECK VALVE

19

16        16

15

14        14

EP 1 257 301 B1

FIG. 2.

EP 1 257 301 B1

FIG.3.

9

FIG. 4.